# EUROPÄISCHE PATENTSCHRIFT

(11) **EP 1 603 481 B1**
(45) Veröffentlichungstag und Bekanntmachung des Hinweises auf die Patenterteilung: **23.01.2008**
(21) Anmeldenummer: 03799440.7
(22) Anmeldetag: 31.12.2003
(51) Int. Cl.: A61C 13/00, A61C 8/00

(54) **VERFAHREN ZUR AUTOMATISCHEN ERZEUGUNG EINER DENTALEN SUPRASTRUKTUR ZUR VERBINDUNG MIT EINEM IMPLANTAT**
METHOD FOR AUTOMATICALLY CREATING A DENTAL SUPERSTRUCTURE FOR JOINING TO AN IMPLANT
PROCEDE DE PRODUCTION AUTOMATIQUE D'UNE SUPERSTRUCTURE DENTAIRE POUR ASSURER LA JONCTION AVEC UN IMPLANT

(30) Priorität: 02.01.2003 DE 10300301
(43) Veröffentlichungstag der Anmeldung: 14.12.2005
(73) Patentinhaber: Sirona Dental Systems GmbH, 64625 Bensheim (DE)
(72) Erfinder: SALIGER, Günter, 64625 Bensheim (DE); ROTHENBERGER, Bernd, 76593 Gernsbach (DE); PIEPER, Reinhard, 64625 Bensheim (DE)
(74) Vertreter: Sommer, Peter
(86) Internationale Anmeldenummer: PCT/DE2003/004252
(87) Internationale Veröffentlichungsnummer: WO 2004/060197

(56) Entgegenhaltungen:
- EP-A- 1 062 916
- WO-A-03/024352
- US-A- 6 126 445
- US-A1- 2002 090 592

## Beschreibung

### Technisches Gebiet

Die Erfindung betrifft ein Verfahren zur automatischen Erzeugung einer dentalen Suprastruktur, insbesondere eines Abutments mit einer Anschlussgeometrie für ein dentales Implantat. Die Suprastruktur kann dabei ein- oder mehrteilig sein. Bei mehrteiligen Suprastrukturen für die Versorgung von dentalen Implantaten dient ein Teil der Suprastruktur, nämlich das Abutment, sowohl dem biomechanischen wie dem ästhetischen Zweck, einen Ausgleich der Winkeldifferenz zwischen Implantatachse und Okklusalrichtung herbeizuführen, um bei ästhetisch sinnvollen Lösungen eine gute Übertragung der Kaukräfte auf das Implantat zu gewährleisten.

Die Aufgabe eines Abutments besteht unter anderem darin, auf der dem Kiefer zugewandten Seite den passgenauen Anschluss an das Implantat zu gewährleisten und auf der in die Mundhöhle gerichteten Seite eine Form bereitzustellen, die mit konventioneller Prothetik versorgt werden kann. Insbesondere durch die zweite Anforderung hat sich ein stumpfartiges Aussehen der der Mundhöhle zugewandten Seite des Abutments herausgebildet. Der Stumpf berücksichtigt die Zahnachse des zu ersetzenden Zahns. Diese Achse ist besonders bei Molaren (Backenzähne) senkrecht zur Okklusalfläche der Zähne angeordnet.

### Stand der Technik

Die Formen individueller Abutments wurden bisher vom Zahntechniker oder vom behandelnden Zahnarzt festgelegt.

Im bekannten Stand der Technik werden dabei nur Standard-Abutments verwendet. Um den Winkel zwischen Implantatachse und Okklusion auszugleichen, sind allerdings Standard-Abutments mit festen Kippwinkeln auf dem Markt erhältlich.

Das in der EP 1 062 916 A2 vorgestellte Verfahren beruht darauf, dass in einen konventionellen Abdruck ein sogenanntes Manipulier-Implantat eingebracht wird und damit auf dem Modell eine Situation hergestellt wird, wie sie im Mund des Patienten nach dem Einbringen des Implantats vorliegt. Diese klinische Situation wird dann mit Hilfe eines Scanners vermessen mit dem Ziel, Abutments und gegebenenfalls einen dazugehörigen zweiten Teil der Suprastruktur herzustellen. Zur Vermessung wird ein Hilfselement verwendet. Mit diesem Verfahren werden die Arbeiten, die der Zahntechniker auch heute schon nach dem Stand der Technik ausführen muss, unter Einsatz eines Rechners weitergebildet, d.h. basierend auf einem digitalisierten 3D-Modell werden die auszuführenden Zwischenstufen Modellation von Abutment, Gerüst und Verblendung im Rechner ausgeführt, um schließlich die benötigten Suprastrukturen mit Hilfe einer rechnergesteuerten Schleifmaschine herzustellen. Man spricht hier von einem CAD/CAM-Prozess.

Aus der US 5 989 029 und aus der US 6 231 342 ist bekannt, aus mehreren Vermessungen in unterschiedlichen Richtungen durch Veränderung eines Standard-Abutments ein individuelles Abutment zu berechnen. Allerdings ist auch hier nach wie vor noch die Erstellung eines Abdrucks erforderlich.

Dokument US 6 126 445 offenbart ein Verfahren gemäß dem Oberbegriff von Anspruch 1.

Die Aufgabe der Erfindung besteht darin, dass die Form des individuellen Abutments unter Berücksichtigung der gegebenen Randbedingungen automatisch erstellt werden soll.

Für die Funktion und Ästhetik der Suprastruktur ist dabei neben der äußeren Gestaltung der Krone unter Berücksichtigung der Okklusion auch die Festlegung des Ausgleichswinkels zwischen Implantat- und Zahnachse von Bedeutung.

### Darstellung der Erfindung

Die Aufgabe der Erfindung wird mit dem Verfahren gemäß Anspruch 1 gelöst. Das Verfahren zur automatischen Erzeugung einer mehrteiligen Suprastruktur, insbesondere eines Abutments mit einer Krone, zur Verbindung mit einem Implantat anhand einer digitalen Modellbeschreibung der Form umfasst ein Erfassen der klinischen Situation oder einer gestalteten klinischen Situation des Implantats als digitale Daten, eine Analyse dieser Situation und Bestimmung der Implantatachse, eine Berechnung der optimalen Form der Suprastruktur, und ein Herstellen der Einzelteile mit einer Bearbeitungsmaschine aus einem oder mehreren Rohlingen aus einem beliebigem Material anhand der digitalen Daten.

Die klinische Situation entspricht der tatsächlichen Situation im Patientenmund. Die gestaltete klinische Situation unterscheidet sich dadurch, dass Maßnahmen ergriffen wurden wie Modellierung des Zahnfleischs durch Aufwachsen oder Festlegen eines Zahnfleischverlaufs anhand eines Datensatzes.

Hierdurch ist es möglich, bei der Berechnung der Außenkontur der zweiteiligen Suprastruktur auch bereits die Verbindungsstelle zwischen dem ersten Teil und dem zweiten Teil einzubeziehen. Als erstes Teil kommt dabei insbesondere ein Abutment, als zweites Teil eine Krone in Betracht. Bei dem erfindungsgemäßen Verfahren wird die Trennung der Suprastruktur in Abutment und Krone und die Formgebung des Abutments automatisch vorgenommen. Dabei wird die optimale individuelle Form des Abutments unter Berücksichtigung von geometrischen, klinischen, materialtechnischen und fertigungstechnischen Aspekten automatisch erzeugt.

In der Regel werden die verschiedenen Teile der Suprastruktur aus unterschiedlichen Rohlingen hergestellt. Unter Umständen ist es aber möglich, einen einzigen Rohling für die Herstellung des ersten und zweiten Teils der Suprastruktur zu verwenden.

Gemäß einer Weiterbildung wird eine Passung zwischen dem digital vorliegenden ersten Teil der Suprastruktur einerseits und dem digital vorliegenden zweiten Teil der Suprastruktur andererseits bestimmt und bei der Herstellung berücksichtigt.

Vorteilhafterweise ist die übrige Form der Suprastruktur durch mindestens zwei der nachfolgenden Eigenschaften Schulterbreite, Neigungswinkel der Suprastruktur gegenüber der Längsachse des Implantats, Drehwinkel der Suprastruktur um die Längsachse im Rohling und Stumpfhöhe beschrieben.

Der Verlauf des Randes der Suprastruktur ist derart, dass er innerhalb des verwendeten Rohlings liegt. Als Rand wird dabei die Trennlinie zwischen einem oberen Teil der Suprastruktur, beispielsweise einer Krone und einem unteren Teil der Suprastruktur, beispielsweise einem Abutment bezeichnet. Dieser Rand sollte aus ästhetischen Gründen auf dem oder unterhalb des Zahnfleischrands liegen. Der Rand der Suprastruktur wird der von einer vorgegebenen klinischen Situation oder aus einer gestalteten klinischen Situation gewonnen.

Unter dentaler Suprastruktur werden alle Bauteile verstanden, die direkt auf einem Implantat befestigt werden können. Dies sind insbesondere Abutments, aber auch Teleskope und anderes.

Die Beschreibung der Form oder des Randes erfolgt dabei in Form digitaler Daten als Linie, Flächen oder Punktwolken, aber auch parametrisch. Die Form der gesamten Suprastruktur kann ebenfalls digital dargestellt werden als bestehend aus Randlinien, als Oberfläche, als Punktwolke, und/oder als parametrische Beschreibung. Beispielhaft ist hier die Darstellung mit einer Randlinie und einem Parametersatz gewählt.

Gemäß einer Weiterbildung wird die Form eines Abutments optimiert bezüglich einer oder mehrerer oder aller der folgenden Parameter, nämlich Mindestmass für die Schulterbreite, maximale Stumpfhöhe begrenzt durch Neigungswinkel gegenüber der Okklusalrichtung, Blockgeometrie und

Höhe der Okklusalfläche, wobei die maximale Stumpfhöhe so bemessen ist, dass sie um ein Höchstmass unterhalb der Höhe der Okklusalfläche liegt, minimale Stumpfhöhe, welche durch die Lage des Kopfes einer Okklusalschraube begrenzt ist, Drehwinkel des Abutments um die Längsachse im Rohling, der sich aus der relativen Lage des Implantats in der klinischen Situation ergibt.

Diese Optimierung erfolgt automatisch anhand der vorliegenden digitalen Daten durch eine geeignete Software und das Ergebnis sind digitale Daten, die das herzustellende Abutment beschreiben. Diese Daten können auf geeignete Weise zu Kontrollzwecken sichtbar gemacht werden, beispielsweise durch Anzeige eines dreidimensionalen Modells auf einem Bildschirm.

Vorteilhafterweise ist die Form des Rohlings und die Form der dentalen Suprastruktur im Koordinatensystem der Anschlussgeometrie an das Implantat beschrieben. Die Anschlussgeometrie an das Implantat ist eine fixe Geometrie, die sowohl im Implantat als auch im Rohling und in der herzustellenden Suprastruktur zu finden ist und in großer Fertigungsgenauigkeit vorliegt. Durch diesen Umstand entfällt eine Umrechnung zwischen verschiedenen Koordinatensystemen bei deren geschickter Wahl. Allgemein reicht aber bereits irgendein bekanntes Koordinatensystem aus, welches beispielsweise durch die Vermessung festgelegt wird.

Vorteilhafterweise kann in besonders problematischen, d.h. uneindeutigen Fällen die Bestimmung der Implantatachse interaktiv mit dem Benutzer erfolgen.

Weitere Ausgestaltungen des Verfahrens sind in den Unteransprüchen wiedergegeben.

### Kurzbeschreibung der Zeichnung

Das erfindungsgemäße Verfahren wird anhand der Zeichnung erläutert. Es zeigt die
- Fig. 1: ein Schemabild für eine zweiteilige Suprakonstruktion für ein dentales Implantat, die
- Fig. 2a,b: eine klinische Situation mit zwei im Kiefer eingebetteten Implantaten, die
- Fig. 3: die Anordnung eines herzustellenden Abutments in einem Rohling, die
- Fig. 4: eine klinische Situation während der Vermessung, die
- Fig. 5: eine Berechnung der Suprastruktur für die klinische Situation gemäß Fig. 4, die
- Fig. 6a-d: Beispiele für die Zerlegung der Suprastruktur in mehrere Teile, die
- Fig. 7a,b: ein Ausführungsbeispiel für Konuskronen in zwei Ansichten.

### Ausführungsbeispiel

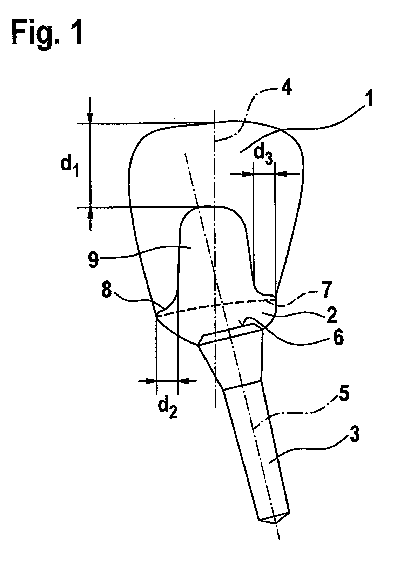

In Fig. 1 ist eine aus einer Krone 1 und einem Abutment 2 bestehende zweiteilige Suprastruktur eines Implantats 3 schematisch dargestellt. Die Einschubrichtung der Krone 1 wird durch eine Achse 4 angedeutet.

Die Lage des Implantats 3 im Kieferknochen wird durch eine weitere Achse 5 dargestellt. Die Achsen 4 und 5 sind nur in seltenen Fällen übereinstimmend, da die Einbaulage des Implantats innerhalb der klinischen Situation patientenabhängig ist und unter Berücksichtigung einerseits des Knochenangebots und andererseits der vorhandenen Zähne festzulegen ist. Diese Größen sind entscheidend für die fachgerechte Position und Orientierung des Implantats hinsichtlich der späteren mechanischen Festigkeit beim Kauen. Die Lage des Implantats mit seiner Achse 5 kann deshalb von der Lage der ursprünglichen Zahnwurzel abweichen.

Das Abutment 2 ist über eine Anschlussgeometrie 6 mit dem auf Kieferknochen-Niveau befindlichen Ende des Implantats 3 unter Ausbildung eines Formschlusses verbunden. Die ideale Abutmentform erweitert sich von der Anschlussgeometrie zum Implantat ausgehend so, dass sie ca. 1 mm unterhalb des Zahnfleischs (Gingiva) mit dem Abutmentrand 7 einen Zahnquerschnitt formt.

Oberhalb des Abutmentrandes 7 formt sie eine umlaufende Schulter 8, auf der sich die Krone 1 abstützt. Diese Schulter 8 ist breit genug, um sicherzustellen, dass die Wandstärke der Krone den materialbedingten Mindestwert nicht unterschreitet. Die Schulter 8 geht über in einen schlankeren Stumpf 9.

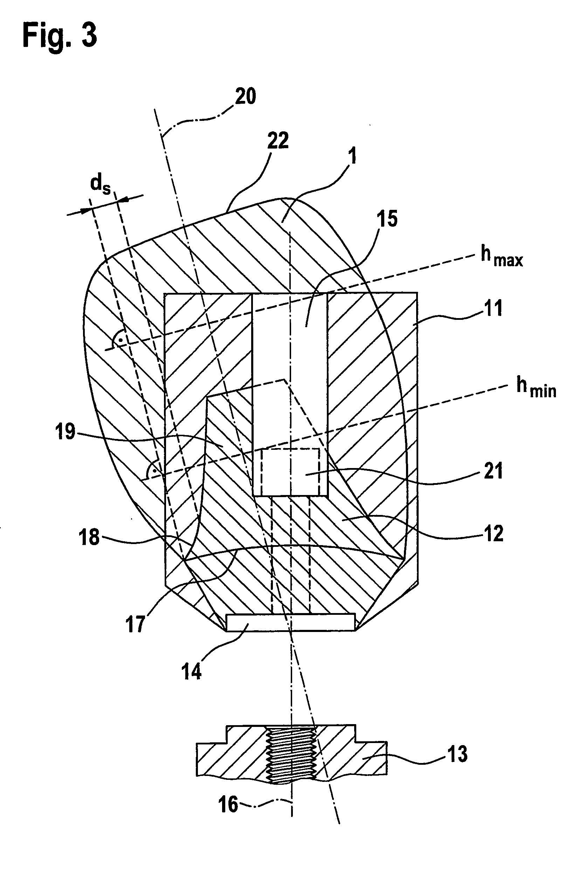

Dieser Stumpf 9 zeigt in die durch die Achse 4 angegebene Einschubrichtung. Im idealen Fall gleicht das Abutment 2 durch seine Form den als Kippwinkel bekannten Winkel zwischen der Achse 5 des Implantats und der Einschubrichtung derart aus, dass die Krone 1 in okklusaler Richtung entlang der Achse 4 aufgesetzt werden kann (siehe hierzu Fig. 3).

Anstelle der Okklusalrichtung kann auch eine beliebige andere Achse herangezogen werden, etwa eine gemeinsame Einschubachse des oberen Teils der zweiteiligen Suprastruktur bei einer Suprastruktur für mehrgliedrigen Restaurationen.

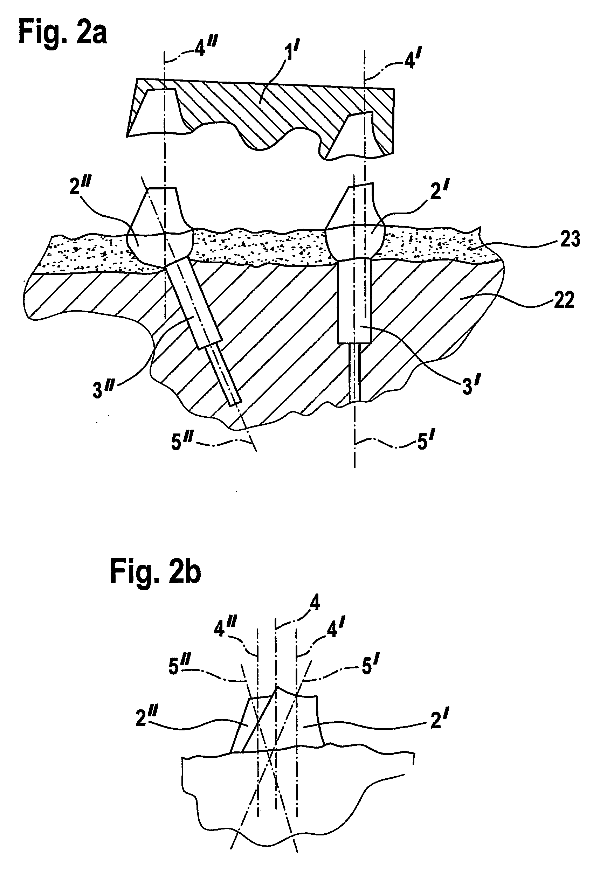

In den Fig. 2a,b ist eine klinische Situation mit zwei im Kiefer eingebetteten Implantaten im Längsschnitt dargestellt, wobei anstelle der Krone ein Gerüst 1' auf zwei Abutments 2', 2" aufgesetzt wird. Die Implantate 3', 3" weisen Achsen 5', 5" auf die zueinander windschief sein können (dargestellt in Fig. 2b). Die Abutments 2', 2" weisen jedoch Einschubachsen 4', 4" auf, die zueinander parallel sind. Die Implantate 3', 3" sind im Kieferknochen 22 verankert und erstrecken sich bis zum Zahnfleisch 23.

Ein ideales Abutment weist außerdem einen als "Verdrehschutz" ausgebildeten Querschnitt auf, der verhindert, dass sich die aufgesetzte Krone gegen das Abutment verdrehen lässt.

Die äußere Geometrie des Abutments trägt den Mindestgrößen d₁, d₂ und d₃ Rechnung, die später erläutert werden.

In Fig. 3 ist ein aus einem Rohling 11 herzustellendes Abutment 12 schematisch dargestellt. Der Rohling 11 weist zur formschlüssigen Verbindung mit einem Implantat 13 eine Anschlussgeometrie 14 auf, wobei das Abutment 12 so in den Rohling 11 gelegt ist, dass es die Anschlussgeometrie 14 übernimmt.

Der Rohling 11 enthält eine Bohrung 15 zur Aufnahme einer Befestigungsschraube an dem Implantat. Die Bohrung 15 ist im Ausführungsbeispiel konzentrisch zu einer Mittelachse 16 des Implantats 13. Die Mittelachse 16 des Implantats 13 gibt aber hauptsächlich die Richtung der Befestigung an der Anschlussgeometrie 14 vor.

Der Rohling 11 ist im Bereich der Anschlussgeometrie konisch ausgebildet, so dass er die Formgebung des Abutments zu einem Abutmentrand 17 hin vorzeichnet. An den Abutmentrand 17 schließt sich eine Schulter 18 an, die in einen Stumpf 19 übergeht.

Der Stumpf 19 ist derart in dem Rohling 11 angeordnet, dass eine die Richtung des Aufsetzens einer Krone 1 wiedergebende Achse 20 die Mittelachse 16 in einem Winkel schneidet. Grundsätzlich können die Achsen 16 und 20 auch gegeneinander windschief sein, es hat sich jedoch gezeigt, dass die Verwendung von sich schneidenden Achsen hinreichend ist.

Dabei ist sichergestellt, dass der gestrichelt dargestellte Kopf 21 einer Okklusalschraube innerhalb der Bohrung 15 liegt, die von der Wand des Abutments 12 begrenzt ist.

Die optimale Abutmentform variiert die ideale Abutmentform derart, dass die nachfolgend beschriebenen Randbedingungen, nämlich klinische Situation, geometrische, materialtechnische und fertigungstechnische Bedingungen erfüllt sind.

Die klinische Situation bestimmt die ideale Abutmentform. Neben Lage und Orientierung des dentalen Implantats wird die umgebende Zahnsituation analysiert. Aus dieser Analyse ergibt sich die Richtung der Okklusion und damit der Winkel, den das Abutment ausgleichen soll. Außerdem wird die Höhe des Okklusaltableaus ermittelt.

Die Rohlinge, in der Regel Keramikblöcke, die für die Herstellung von Abutments verwendet werden, weisen bestimmte geometrische Randbedingungen auf. Aus Präzisionsgründen ist bei ihrer Herstellung die Größe und Form dieser Blöcke sehr begrenzt variierbar. Aus diesen Blöcken kann nicht jede beliebige ideale Abutmentform hergestellt werden. Unter anderem ist zu berücksichtigen, dass der Kopf 21 der Okklusalschraube komplett innerhalb der Form des Abutments 12 verschwinden soll, siehe Fig. 3.

Die Materialstärke des Werkstoffes und insbesondere einer Keramik für das Abutment 2 und die darauf passende Krone 1 darf bestimmte Mindestwerte d₁, d₂ und d₃ nicht unterschreiten. Diese Wandstärken sind aber stark materialabhängig und können daher für jedes Material gesondert berücksichtigt werden.

Sind zum Zeitpunkt der Formgebung des Abutments bereits fertigungstechnische Randbedingungen bekannt, die aus der Herstellungsweise von Abutment und Krone herrühren, können auch sie einen Einfluss auf die optimale Form des Abutments haben.

Ist z.B. bekannt, dass beide Keramikformen in einer Schleifmaschine mit begrenzten Freiheitsgraden für Instrumente hergestellt werden, kann bei der Generierung der optimalen Form sichergestellt werden, dass die Form des Abutments überhaupt geschliffen werden kann. Das gilt nicht nur für die Positivform des Abutments, sondern auch für die Negativform, also für die Innenseite der Krone, welche eine Befestigungsfläche darstellt.

Um die ideale und optimale Form eines Abutments automatisch zu erzeugen, muss zunächst eine Modellbeschreibung der Form vorliegen. Dabei kann z.B. der Abutmentrand als Linie im Koordinatensystem der Anschlußgeometrie des Implantats, die übrige Form als Parameter beschrieben sein.

Die Linie definiert den Übergang vom Abutment zur Krone und ist räumlich geschlossen. Sie liegt z.B. als Liste von Punkten oder als Funktion vor. Diese Linie verläuft idealerweise aus ästhetischen Gründen etwas unterhalb des umlaufenden Zahnfleisches.

Als Parameter kommen Schulterbreite, Kippwinkel, Drehwinkel des Abutments um die Längsachse im Rohling und Stumpfhöhe in Frage.

Der Rohling lässt sich ebenfalls im Koordinatensystem seiner Anschlussgeometrie, die perfekt auf die Anschlussgeometrie des Implantats passt, beschreiben. Die Form des idealen Abutmentrandes kann dann stückweise lokal angepasst werden, so dass diese Linie gänzlich innerhalb der Außenkontur des verwendeten Rohlings 11 liegt.

Um das gesamte Abutment zu beschreiben, müssen dann lediglich alle Parameter auf alle Randbedingungen hin optimiert werden. Die Schulterbreite dₛ beträgt bei den derzeit verwendeten üblichen Keramiken mindestens 1 mm. Die maximale Stumpfhöhe hₘₐₓ ist durch den Kippwinkel, die Blockgeometrie und die Höhe des Okklusaltableaus 22 begrenzt und liegt mindestens 1 mm unterhalb dieser Höhe. Die minimale Stumpfhöhe hₘᵢₙ ist durch die Lage des Kopfes 21 der Okklusalschraube nach unten begrenzt.

Die minimale Höhe hₘᵢₙ und die maximale Höhe hₘₐₓ liegen senkrecht zu der Achse 20, welche die Richtung des Aufsetzens der Krone 1 auf den Stumpf 19 wiedergibt. Die minimale Schulterbreite dₛ ist parallel zu der Achse 20 zu messen.

Der Drehwinkel des Abutments um die Längsachse im Rohling ergibt sich aus der relativen Lage des Implantats in der klinischen Situation und ist wegen der Rotationssymmetrie des Rohlings durch diesen nicht weiter begrenzt.

Durch Anwendung dieses Verfahrens lässt sich die optimale individuelle Abutmentform automatisch erzeugen und gegebenenfalls maschinell herstellen.

Zunächst wird die klinische Situation oder eine gestaltete klinische Situation des Implantats digital erfasst, z.B. durch eine intraorale Messkamera. Anschließend wird diese Situation unter Berücksichtigung auch der Nachbarzähne und der Lage und der Orientierung des Implantats analysiert und die Implantatachse bestimmt. Dies kann auch interaktiv geschehen. Danach liegen alle Daten vor, die für die automatische Erzeugung einer Abutmentform benötigt werden. Mit Hilfe der oben beschriebenen Randbedingungen wird zunächst die ideale Form und dann davon ausgehend die optimale Form des Abutments berechnet.

Die Aufteilung in zwei Schritte ist dabei nicht zwingend notwendig, insbesondere muss der Anwender das zwischenberechnete Standardabutment nicht angezeigt bekommen. Nur aus programmtechnischer Sicht wird anhand der Randparameter wie Geometrie des Blockes und Kippwinkel der Implantatachse unter Anwendung von Konstruktionsregeln ein Standardabutment berechnet und dann dieses Abutment bezüglich der gewünschten Linie angepasst.

Anschließend kann sich der Anwender der Gestaltung der Krone zuwenden.

Da beide Teile der Suprakonstruktion für das Implantat digital vorliegen, kann eine optimale Passung zwischen ihnen sichergestellt werden. Die Einzelteile können jederzeit mit einer Bearbeitungsmaschine aus beliebigem Material, insbesondere aus Keramik oder Metall, aber auch aus Kunststoff hergestellt werden.

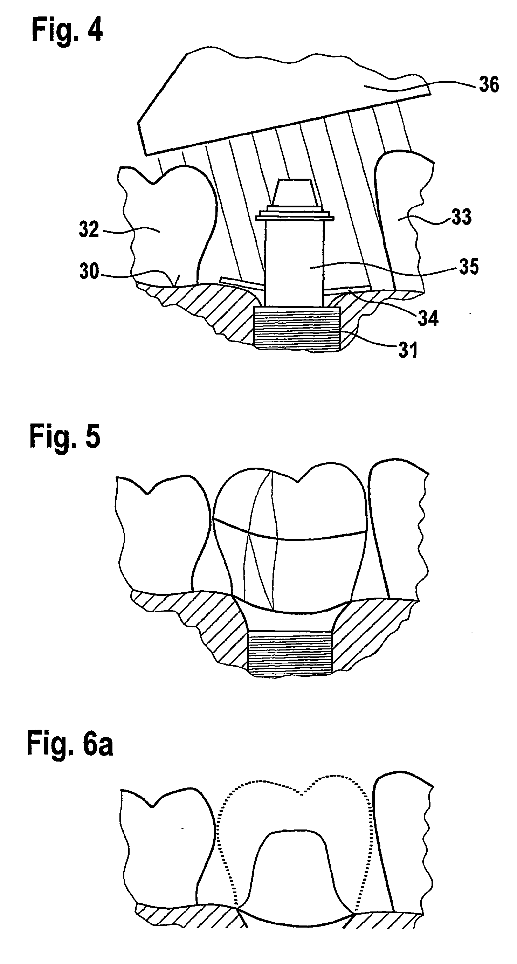

In Fig. 4 ist ein in einem nicht dargestellten Kieferknochen unterhalb eines Zahnfleischrandes 30 eingebrachtes Implantat 31 dargestellt. Zu beiden Seiten des Implantats 31 sind Nachbarzähne 32, 33 vorhanden, welche die seitliche Ausdehnung einer zu konstruierenden Suprastruktur begrenzen. Die in Fig. 4 dargestellte klinische Situation wird dadurch gestaltet, dass im unmittelbaren Bereich des Implantats 31 der tatsächliche Verlauf des Zahnfleischrandes 30 durch ein Ausgleichsteil 34 auf einen gewünschten Verlauf gebracht wird, der zusammen mit einer am Implantat 31 angebrachten Messvorrichtung 35 über eine Intraoral-Kamera 36 vermessen wird. Über das Vermessungsteil 35 lässt sich auf die Lage und Orientierung des Implantats 31 schließen, das Ausgleichsteil 34 legt den Verlauf des Zahnfleisches fest.

Anhand der Vermessungsdaten kann eine in Fig. 5 dargestellte Suprastruktur berechnet werden. Aus der bekannten Okklusalfläche der Nachbarzähne wird die Größe und die Orientierung der für das Implantat benötigten Zahnkrone bestimmt. Die zervikale Bestimmungslinie eines aus einer Zahnbibliothek ausgewählten Büchereizahns wird in mesiodistaler Richtung leicht unter das Niveau gelegt, das durch das Ausgleichsteil 34 gemessen wurde. Zusammen mit der bekannte Lage des Implantatkopfes wird die Zervikalfläche der herzustellenden Suprastruktur berechnet.

In Fig. 6a wird die Zerlegung der Suprastruktur in zwei Teile schematisch gezeigt, wobei es sich hier um ein Abutment und eine Krone handelt, die über eine Passung, auch als Trennfläche bezeichnet, verbindbar sind. Die Gestalt dieser Trennfläche kann vom Benutzer in Grenzen variiert werden, solange die übrigen Konstruktionsregeln beachtet werden.

Wesentlich ist, dass in diesem Beispiel die gesamte Suprastruktur berechnet wird und auch die Trennfläche automatisch festgelegt wird. Diese Trennfläche gestattet es dem Anwender, die Suprastruktur aus mehr als einem Körper herzustellen. Dies kann angebracht sein, weil unterschiedliche Restaurationsmaterialien mit unterschiedlichen Merkmalen zum Einsatz kommen sollen bzw. kommen müssen, weil eine ästhetisch hochwertigere Gestaltung erforderlich ist oder weil die geometrischen Verhältnisse keine andere Lösung zulassen.

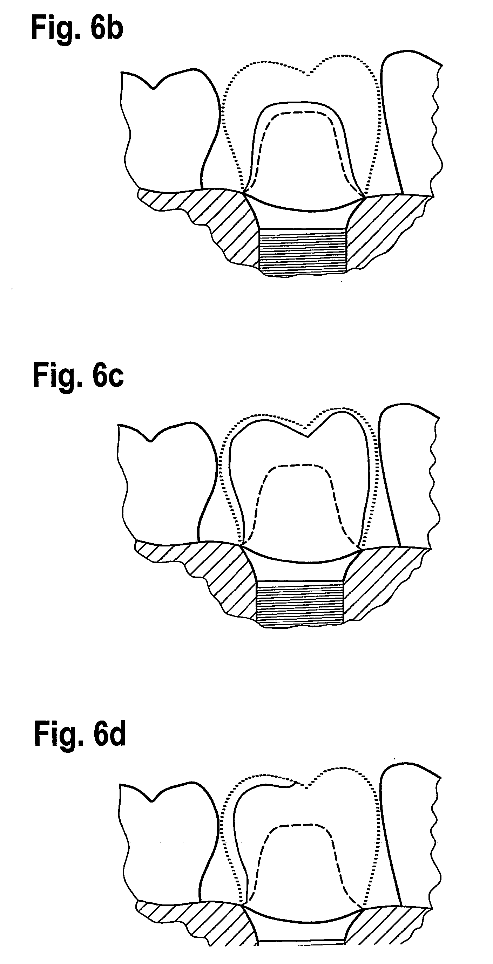

In Fig. 6b ist die Zerlegung der Suprastruktur in ein Käppchen und ein entsprechendes Abutment gezeigt, wobei das Käppchen konventionell mit Keramiken verblendet wird.

In Fig. 6c ist die Zerlegung der Suprastruktur in eine Reduzierkrone und ein Abutment der endgültigen Versorgung dargestellt. Durch die reduzierte Größe liegt diese Krone außerhalb der Okklusion des gegenüberliegenden Zahns und erfährt daher keine Krafteinwirkung außer einer Restkraft beim Kauen. Dabei kann von dem Anwender die tatsächlich notwendige und funktional korrekte Krone konstruiert werden. Diese Krone wird dann rechnerisch so reduziert, das sie sicher außerhalb der Okklusion ist. Dieses Werkstück kann dann z. B. aus Kunststoff hergestellt werden. In einer zweiten Sitzung kann dann eine Krone gemäß dem Orginaldatensatz ausgeschliffen und die endgültige Krone kann eingesetzt werden.

In Fig. 6d ist eine Zerlegung der Suprastruktur in eine teilweise verblendbare Krone und ein Abutment gezeigt. Dabei kann sowohl die Passung zwischen Abutment und Krone als auch die Passung zwischen Krone und Verblendung unter Berücksichtigung der Konstruktionsregeln automatisch berechnet werden.

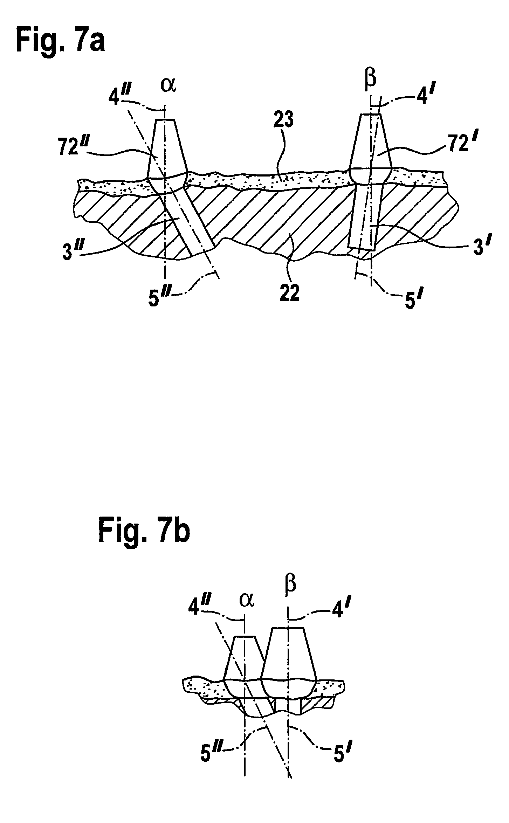

Sollen ein oder mehrere Implantate mit Teleskopen oder mit Konuskronen versorgt werden, gibt es prinzipiell zwei unterschiedliche Vorgehensweisen. Das Implantat, oder auch mehrere Implantate, wird mit einem normalen Abutment versorgt, auf dieses Abutment wird ein Teleskop-Kronengerüst gesetzt, auf das die Prothese aufgeschoben werden kann. Alternativ dazu kann das Abutment selbst als in Fig. 7a und b dargestellte Konuskrone geformt sein. In diesem Fall bildet das Abutment keinen herkömmlichen Kronenstumpf, sondern die Teleskopkrone. Seine Form kann in diesem Fall durch einen anderen Satz Parameter beschrieben werden, z.B. könnte auch hier eine Linie die Randform beschreiben, außerdem definieren Konushöhe und KonusWinkel die Form innerhalb des Randes. Die Implantate 3', 3" weisen Achsen 5', 5" auf, die zueinander windschief sein können (dargestellt in Fig. 7a). Die Konuskronen 72', 72" weisen jedoch Einschubachsen 4', 4" auf, die zueinander parallel sind. Die Implantate 3', 3" sind im Kieferknochen 22 verankert und erstrecken sich bis zum Zahnfleisch 23. Die Unterteilung in Abutment und Krone oder jede andere Art der Unterteilung wird dann automatisch berechnet.

Die Regeln, die es bei der Gestaltung des Abutments zu beachten gilt, sind im Rechner abgebildet und kommen bei der automatischen Aufspaltung in Abutment und Suprastruktur zur Anwendung.

Gemäß der EP 1 062 916 A2 wird sowohl für die Formgebung des Abutments wie die des Gerüsts und der Verblendung entweder ein Wax-Up oder eine Bibliothek verwendet. Verwendet man einen Wax-Up bedeutet das, dass mehrere Messaufnahmen ausgeführt werden müssen, um hintereinander die Herstellung von Abutment, Gerüst und Verblendung zu ermöglichen.

Die Erfindung hingegen beschleunigt den gesamten Ablauf und trägt mit dazu bei, die Verlagerung der Technik zum Zahnarzt hin zu ermöglichen.

Da die heutigen Implantate, sofern sie zur Versorgung einzelner Zähne verwendet werden, nicht sofort den Kaukräften ausgesetzt werden dürfen, weil das die innige Verbindung zum Kieferknochen beeinträchtigt, verwenden die Implantatsysteme sogenannte Einheilkappen. Nachdem das Implantat gesetzt wurde, verschließt der Zahnarzt die mechanische Kupplung am Implantat mit einer Einheilkappe und näht das Zahnfleisch wieder komplett zu, um bei einer zweiten Operation nach ca. 8 Wochen den Bereich der Einheilkappe wieder zu eröffnen und dann eine prothetische Versorgung mit Abutment und Suprastruktur einzuleiten. Bei anderen Systemen liegt die Einheilkappe offen und befindet sich auf dem Niveau des Zahnfleischs. Hier wird die Einheilkappe auch dazu genutzt, um das Zahnfleisch so zu formen, so dass sich im Vergleich zu den Nachbarzähnen ein natürlicher Verlauf ergibt.

Die Suprastruktur 1' kann mehrere Abutments aufweisen, die durch eine gemeinsame Gerüstkonstruktion miteinander verbunden sind, Fig. 2a.

## Patentansprüche

1. Verfahren zur automatischen Erzeugung einer dentalen Suprastruktur zur Verbindung mit einem Implantat anhand einer digitalen Modellbeschreibung der Form, umfassend folgende Schritte:
- Erfassen einer tatsächlichen klinischen Situation oder einer gestalteten klinischen Situation des Implantats (3; 13) als digitale Daten;
- Analyse dieser Situation und Bestimmung der Implantatachse (5; 16);
- Berechnen der optimalen Form der Suprastruktur (1, 2);
**gekennzeichnet durch**
- automatisches Trennen der Suprastruktur in einen ersten Teil (1) und einen zweiten Teil (2)
- Herstellen der Einzelteile mit einer Bearbeitungsmaschine aus mindestens einem Rohling (11) anhand der digitalen Daten.

2. Verfahren nach Anspruch 1, **dadurch gekennzeichnet, dass** eine Passung zwischen dem digital vorliegenden ersten Teil (1) der Suprastruktur einerseits und dem digital vorliegenden zweiten Teil (2) der Suprastruktur andererseits bestimmt wird.

3. Verfahren nach Anspruch 1 oder 2, **dadurch gekennzeichnet, dass** die Form des mit dem Implantat zu verbindenden Teils der Suprastruktur beschrieben ist durch mindestens zwei der nachfolgenden Eigenschaften Schulterbreite, Neigungswinkel der Suprastruktur gegenüber der Längsachse (5) des Implantats (3), Drehwinkel der Suprastruktur um die Längsachse (16) im Rohling (11) und Stumpfhöhe.

4. Verfahren nach einem der Ansprüche 1 bis 3, **dadurch gekennzeichnet, dass** ein Teil der Suprastruktur ein Abutment ist und dass die Form eines Abutments (2) optimiert wird bezüglich einer oder mehrerer oder aller der folgenden Parameter:
- ein Mindestmass für die Schulterbreite;
- eine maximale Stumpfhöhe begrenzt durch den Neigungswinkel der Suprastruktur gegenüber der Längsachse (5) des Implantats (3), die Geometrie des Rohlings (11) und die Höhe der Okklusalfläche (22), wobei die maximale Stumpfhöhe so bemessen ist, dass sie um ein Höchstmass unterhalb der Höhe der Okklusalfläche (22) liegt;
- eine minimale Stumpfhöhe, welche durch die Lage des Kopfes einer Okklusalschraube (14) begrenzt ist;
- einen Drehwinkel des Abutments um die Längsachse im Rohling (11), der sich aus der relativen Lage des Implantats (3; 13) in der klinischen Situation ergibt.

5. Verfahren nach einem der Ansprüche 1 bis 4, **dadurch gekennzeichnet, dass** die Form des Rohlings (11) und die Form der dentalen Suprastruktur (1, 2) im Koordinatensystem der Anschlussgeometrie (6; 14) an das Implantat (3; 13) beschrieben sind.

6. Verfahren nach einem der Ansprüche 1 bis 5, **dadurch gekennzeichnet, dass** die Bestimmung der Implantatachse (5; 16) interaktiv mit dem Benutzer erfolgt.

7. Verfahren nach einem der Ansprüche 1 bis 6, **dadurch gekennzeichnet, dass** ein Teil der Suprastruktur ein Abutment ist und ein weiterer Teil der Suprastruktur eine Krone ist.

8. Verfahren nach einem der Ansprüche 1 bis 6, **dadurch gekennzeichnet, dass** ein Teil der Suprastruktur ein Abutment ist und ein weiterer Teil der Suprastruktur ein Käppchen ist.

9. Verfahren nach einem der Ansprüche 1 bis 6, **dadurch gekennzeichnet, dass** ein Teil der Suprastruktur ein Abutment ist und ein weiterer Teil der Suprastruktur eine reduzierte Krone ist.

10. Verfahren nach einem der Ansprüche 1 bis 6, **dadurch gekennzeichnet, dass** die Suprastruktur dreiteilig ist und ein erstes Teil der Suprastruktur ein Abutment ist und ein zweites Teil der Suprastruktur eine teilweise verblendete Krone ist und dass der dritte Teil ein Verneer ist und dass neben der Passung des ersten und zweiten Teils auch eine Passung für das dritte Teil mit dem ersten Teil und/oder mit dem zweiten Teil berechnet wird.

11. Verfahren nach einem der Ansprüche 1 bis 6, **dadurch gekennzeichnet, dass** die Suprastruktur (1') mehrere Abutments aufweist, die durch eine gemeinsame Gerüstkonstruktion miteinander verbunden sind.

12. Verfahren nach einem der Ansprüche 1 bis 11, **dadurch gekennzeichnet, dass** die Aufteilungsregeln vom Benutzer variierbar sind.

13. Verfahren nach einem der Ansprüche 1 bis 12, **dadurch gekennzeichnet, dass** das mit dem Implantat verbundene Teil der Suprastruktur in der endgültigen Größe berechnet wird und dass der mit diesem Teil verbundene weitere Teil der Suprastruktur als provisorische Suprastruktur mit gegenüber den endgültigen Außenabmessungen verringerten Außenabmessungen unter Beibehaltung der Passung berechnet wird.

14. Verfahren nach Anspruch 13, **dadurch gekennzeichnet, dass** unter Verwendung desselben Datensatzes der Teil der Suprastruktur in den endgültigen Abmessungen berechnet wird.

## Claims

1. A method of automatically fabricating a suprastructure to be attached to an implant with the help of a digital model description of the shape, comprising the following steps:
• recording a real clinical situation or a shaped clinical situation of the implant (3; 13) as digital data,
• analyzing this situation and determining the implant axis (5; 16), and
• computing the optimum shape of the suprastructure (1, 2),
**characterized by** the following steps:
• automatic separating of the suprastructure into a first element (1) and a second element (2) and
• fabricating the individual elements from one or more blanks (11) on the basis of said digital data by means of machining equipment.

2. A method as defined in claim 1, **characterized in that** a mating surface between the digitized first element (1) of the suprastructure on the one hand and the digitized second element (2) of the suprastructure on the other hand, is determined.

3. A method as defined in claim 1 or claim 2, **characterized in that** the shape of that element of the suprastructure which is to be connected to the implant is described by at least two of the following parameters: the shoulder width, the tilt angle of the suprastructure relative to the longitudinal axis (5) of said implant (3), the angle of rotation of the suprastructure about the longitudinal axis (16) in said blank (11), and the height of said post.

4. A method as defined in any one of claims 1 to 3, **characterized in that** one element of the suprastructure is an abutment and that the shape of an abutment (2) is optimized with reference to one or more or all the following parameters:
• a minimum value for the shoulder width;
• a maximum height of the post delimited by the tilt angle of the suprastructure relative to the longitudinal axis (5) of said implant (3), the geometry of said blank (11), and the height of the occlusal surface (22), the maximum height of the post being such that it is disposed at a maximum distance below the height of the occlusal surface (22);
• a minimum height of the post delimited by the position of the head of an occlusal screw (14);
• an angle of rotation of the abutment about the longitudinal axis in said blank (11), which is given by the relative position of said implant (3; 13) in the clinical situation.

5. A method as defined in any one of claims 1 to 4, **characterized in that** the shape of said blank (11) and the shape of the dental suprastructure (1, 2) are described in the coordinate system of the geometry (6; 14) for attachment to said implant (3; 13).

6. A method as defined in any one of claims 1 to 5, **characterized in that** determination of the axis of said implant (5; 16) is effected interactively by the user.

7. A method as defined in any one of claims 1 to 6, **characterized in that** one element of the suprastructure is an abutment and a further element of the suprastructure is a crown.

8. A method as defined in any one of claims 1 to 6, **characterized in that** one element of the suprastructure is an abutment and a further element of the suprastructure is a cap.

9. A method as defined in any one of claims 1 to 6, **characterized in that** one element of the suprastructure is an abutment and a further element of the suprastructure is a reduced crown.

10. A method as defined in any one of claims 1 to 6, **characterized in that** the suprastructure comprises three elements, and a first element of the suprastructure is an abutment and a second element of the suprastructure is a partially veneered crown and the third element is a veneer, and that not only the mating surface between said first and second elements but also a mating surface between said third element and said first element and/or said second element is/are computed.

11. A method as defined in any one of claims 1 to 6, **characterized in that** said suprastructure (1') comprises a number of abutments which are interconnected by a common frame construction.

12. A method as defined in any one of claims 1 to 11, **characterized in that** the distribution rules can be varied by the user.

13. A method as defined in any one of claims 1 to 12, **characterized in that that** element of the suprastructure which is connected to the implant is computed in its final size and that the further element of the suprastructure connected to this element is computed as a provisional suprastructure having exterior dimensions which are smaller than the final exterior dimensions while retaining the mating surface.

14. A method as defined in claim 13, **characterized in that** the same data set is used to compute said element of the suprastructure with its final dimensions.

## Revendications

1. Procédé de fabrication automatique d'une superstructure dentaire destinée à être assemblée à un implant, à l'appui d'une description de modèle numérique de la forme, comportant les étapes suivantes :
- enregistrement d'une situation clinique réelle ou d'une situation clinique créée de l'implant (3 ; 13) sous forme numérique ;
- analyse de cette situation et détermination de l'axe de l'implant (5 ; 16) ;
- calcul de la forme optimale de la superstructure dentaire (1, 2) ;
**caractérisé par**
- une séparation automatique de la superstructure en une première partie (1) et une deuxième partie (2) ;
- fabrication des parties séparées au moyen d'une machine d'usinage à partir d'au moins une ébauche (11) à l'appui des données numériques.

2. Procédé selon la revendication 1, **caractérisé en ce qu'**un ajustement est déterminé entre la première partie (1) de la superstructure disponible sous forme numérique, d'une part, et la deuxième partie (2) de la superstructure disponible sous forme numérique, d'autre part.

3. Procédé selon la revendication 1 ou 2, **caractérisé en ce que** la forme de la partie de la superstructure à assembler à l'implant est définie par au moins deux des propriétés suivantes, à savoir la largeur d'épaulement, l'angle d'inclinaison de la superstructure par rapport à l'axe longitudinal (5) de l'implant (3), l'angle de rotation de la superstructure autour de l'axe longitudinal (16) dans l'ébauche (11) et la hauteur du moignon.

4. Procédé selon l'une quelconque des revendications 1 à 3, **caractérisé en ce qu'**une partie de la superstructure est un pilier et **en ce que** la forme d'un pilier (2) est optimisée par rapport à un ou à plusieurs des paramètres suivants ou par rapport à tous les paramètres suivants :
- une dimension minimum pour la largeur d'épaulement ;
- une hauteur maximale du moignon délimitée par l'angle d'inclinaison de la superstructure par rapport à l'axe longitudinal (5) de l'implant (3), la géométrie de l'ébauche (11) et la hauteur de la surface occlusale (22), la hauteur maximale du moignon étant choisie de telle sorte qu'elle se situe selon une valeur maximale en dessous de la hauteur de la surface occlusale (22) ;
- une hauteur minimale du moignon, qui est délimitée par la position de la tête d'une vis occlusale (14) ;
- un angle de rotation du pilier autour de l'axe longitudinal dans l'ébauche (11), lequel résulte de la position relative de l'implant (3; 13) dans la situation clinique.

5. Procédé selon l'une quelconque des revendications 1 à 4, **caractérisé en ce que** la forme de l'ébauche (11) et la forme de la superstructure dentaire (1, 2) sont décrites dans un système de coordonnées de la géométrie d'assemblage (6 ; 14) sur l'implant (3; 13).

6. Procédé selon l'une quelconque des revendications 1 à 5, **caractérisé en ce que** la détermination de l'axe de l'implant (5 ; 16) est effectuée de manière interactive avec l'utilisateur.

7. Procédé selon l'une quelconque des revendications 1 à 6, **caractérisé en ce qu'**une partie de la superstructure est un pilier et une autre partie de la superstructure est une couronne.

8. Procédé selon l'une quelconque des revendications 1 à 6, **caractérisé en ce qu'**une partie de la superstructure est un pilier et une autre partie de la superstructure est une coiffe.

9. Procédé selon l'une quelconque des revendications 1 à 6, **caractérisé en ce qu'**une partie de la superstructure est un pilier et une autre partie de la superstructure est une couronne réduite.

10. Procédé selon l'une quelconque des revendications 1 à 6, **caractérisé en ce que** la superstructure est réalisée en trois parties, et une première partie de la superstructure est un pilier et une deuxième partie de la superstructure est une couronne enrobée partiellement et **en ce que** la troisième partie est une facette (verneer) et **en ce que**, outre l'ajustement de la première et de la deuxième partie, il est également calculé un ajustement pour la troisième partie avec la première partie et/ou la deuxième partie.

11. Procédé selon l'une quelconque des revendications 1 à 6, **caractérisé en ce que** la superstructure (1') comporte plusieurs piliers, qui sont reliés entre eux par une charpente commune.

12. Procédé selon l'une quelconque des revendications 1 à 11, **caractérisé en ce que** les règles de division peuvent être modifiées par l'utilisateur.

13. Procédé selon l'une quelconque des revendications 1 à 12, **caractérisé en ce que** la partie de la superstructure reliée à l'implant est calculée dans la dimension définitive, et **en ce que** l'autre partie de la superstructure reliée à cette partie est calculée en tant que superstructure provisoire avec des dimensions extérieures inférieures aux dimensions extérieures définitives tout en maintenant l'ajustement.

14. Procédé selon la revendication 13, **caractérisé en ce que** la partie de la superstructure peut être calculée dans ses dimensions définitives moyennant l'utilisation du même jeu de données.
